(19) Europäisches Patentamt
European Patent Office
Office européen des brevets

(11) Publication number: 0 052 436
B1

(12) **EUROPEAN PATENT SPECIFICATION**

(45) Date of publication of patent specification: **15.01.86**

(51) Int. Cl.$^4$: **A 61 K 7/075,** A 61 K 7/08

(21) Application number: **81304974.9**

(22) Date of filing: **22.10.81**

(54) **Shampoo composition.**

(30) Priority: **14.11.80 JP 160357/80**

(43) Date of publication of application:
**26.05.82 Bulletin 82/21**

(45) Publication of the grant of the patent:
**15.01.86 Bulletin 86/03**

(84) Designated Contracting States:
**AT BE CH DE FR IT LI NL**

(56) References cited:
**FR-A-2 438 662**
**US-A-3 806 501**
**US-A-3 957 065**
**US-A-3 961 634**

**TEXTILE PROGRESS, vol. 7, no. 1, 1975, pages 1-70, The Textile Institute, Manchester (GB); N.H. LEON et al.: "The chemical reactivity and modification of keratin fibres"**

(73) Proprietor: **KAO SOAP CO., LTD.**
**1-1, Nihonbashi Kayaba-cho, Chuo-ku**
**Tokyo (JP)**

(72) Inventor: **Matsunaga, Kinjiro**
**Ovaza Wado No 331-21 Miyashiro-machi**
**Minamisaitama-gun Saitama-ken (JP)**
Inventor: **Okumura, Takeo**
**6-22-19 Nakashizu**
**Sakura-shi Chiba-ken (JP)**
Inventor: **Naito, Sachiro**
**4-35-12-303 Kamiyouga**
**Setagaya-ku Tokyo (JP)**
Inventor: **Tsushima, Rikio**
**4-21-302 Akiba-cho**
**Wakayama-shi Wakayama-ken (JP)**

(74) Representative: **Evans, David Charles et al**
**F.J. CLEVELAND & COMPANY 40-43, Chancery Lane**
**London, WC2A 1JQ (GB)**

**EP 0 052 436 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

This invention relates to a shampoo composition and more particularly, to a shampoo composition which comprises a shampoo base containing anionic surface active agents and derivatives of keratin material and which shows an excellent hair conditioning effect and mild eye irritation.

Description of the Prior Art

Hitherto employed shampoos generally comprise singly or in combination, as their base, anionic surface active agents such as alkylsulfates and polyoxyethylenealkylsulfates and the like, nonionic surface active agents such as polyoxyethylene alkyl ethers, fatty acid alkanolamides and the like, or amphoteric surface active agents such as alkylbetaines, alkylamine oxides and the like.

On washing of hair with the shampoos containing these bases, it will be found that the sebum or other oils existing on the hair surface are too extensively washed off, so that the feeling of the washed hair becomes very poor to the touch and hard to comb or brush. In addition, when completely dried, such hair is hard to style. Especially in winter when the humidity is low, since static electricity is readily generated on brushing, and hair fly occurs with the attendant disadvantage that the hair is hard to comb, causing split-ends or broken hair.

In order to overcome the above disadvantage, it is known to add oils to ordinary shampoo bases to supplement the oil at the time of washing. Ordinarily employed shampoos are admixed with a variety of oils.

However, when formulated, the shampoo is emulsified or solubilized by the action of surface active agents, so that it is difficult to add oils in amounts sufficient for scalp and hair without impairing the stability of the shampoo.

With shampoos to which large amounts of oil are added, the amount of oil absorbed on the hair increases but they have the disadvantage that lathering and detergency which shampoos should originally have deteriorate a great deal with the commercial value being considerably impaired.

In recent years, on the other hand, there have been proposed many shampoo compositions including cationic polymers for the purpose of imparting a rinsing effect to hair after washing. However, these compositions have following disadvantages: (1) Having a conditioning effect but a deterioration in lathering ability and detergency; (2) Coloration or discoloration with time; (3) Excellent in lathering characteristic but poor in conditioning effect; and (4) high cost.

In U.S.A.—A—3961 634 there is described a hair treatment composition including an anionic surface active agent and a hydrolytic product of keratin (Keratose) along with hair bleaching agents.

According to the present invention there is provided a shampoo composition comprising a shampoo base containing at least one anionic surface active agent and a hair conditioning derivative of keratin material selected from (1) derivatives of the mercapto groups of the products obtained by reduction of keratin materials, and (2) a mixture thereof with oxidized keratin materials containing —$SO_3H$ groups.

*Detailed description and embodiments of the invention*

Preferable anionic active agents to be used as the base of the shampoo composition include:

(1) Linear or branched alkylbenzenesulfonates whose alkyl group has an average of 10—16 carbon atoms;

(2) Polyoxyalkylenealkylsulfates with an average of 0.5—8 moles of ethylene oxide and/or propylene oxide added per molecule, which have a linear or branched alkyl group having an average of 8—20 carbon atoms,

(3) Alkylsulfates whose alkyl group has an average of 10—20 carbon atoms;

(4) Olefinsulfonates having an average of 10—12 carbon atoms per molecule thereof;

(5) Alkanesulfonates having an average of 10—20 carbon atoms per molecule thereof;

(6) Alkylethoxycarbonates having an average of 10—20 carbon atoms in the alkyl group thereof and with an average of 0.5—8 moles of ethylene oxide added per molecule; and

(7) Derivatives of succinic acid represented by the formula

$$\begin{array}{l} R—CHCOOX \\ \quad\;\;\; | \\ \quad\;\; CH_2COOY \end{array}$$

(in which R represents an alkyl group or alkenyl group having 6—20 carbon atoms and X and Y independently represent a counter ion).

The counter ions of these anionic surface active agents include alkali metal ions such as sodium and potassium, alkaline earth metal ions such as calcium and magnesium and ammonium ions.

Preferable anionic surface active agents among those mentioned above are linear or branched alkylsulfates having an average of 10—16 carbon atoms, polyoxyethylene-alkylsulfates (having an average of 0.5—8 moles ethylene oxide added per molecule) whose alkyl group has an average of 8—20 carbon

atoms, or olefinsulfonates having an average of 10—16 carbon atoms, which are used singly or in combinations.

The derivatives of keratin material to be used in the present invention include derivatives of the mercapto groups of the products obtained by reduction of a keratin which are prepared in the following manner and have an average molecular weight of 30,000—100,000, or a mixture thereof with products obtained by oxidation of a keratin material.

The starting keratin materials include, for example, animal hair, human hair, feathers, claws, horns, hooves and scale, among which wool, hair and feathers are preferably used. These keratin materials may be subjected directly to the oxidation or reduction reaction but, if necessary, may be cut or reduced into pieces of a suitable size or may be pretreated such as by washing and defatting.

(1) Products obtained by oxidation of keratins.

The oxidation of keratin is feasible by a variety of methods known per se (N. H. Leon; Textile Progress, Vol. 7, page 1 (1975)). Oxidizing agents are preferably organic or inorganic agents of the type which electrophilically act on the disulfide bond (S—S bond) in the keratin structure. Examples of the oxidizing agents include organic peracids, inorganic peroxo acids or their salts, permanganic acid or its salts, chromic acid or related compounds, halogens, peroxides and oxyacids or their salts, among which the organic peracids such as peracetic acid, performic acid and perbenzoic acid are preferable.

The oxidation reaction is conducted in liquid media using oxidizing agents in excess with respect to the disulfide bonds in the keratin material, ordinarily in an amount of over two equivalents or more, preferably 4—10 equivalents, of the disulfide bonds. The reaction is preferably conducted under acidic conditions and particularly weakly acidic conditions. The reaction temperature and pressure are varied depending on the type of the oxidizing agent and keratin material and are not critical. Room temperature is generally sufficient but, if necessary, heat be be applied. Atmospheric pressure is satisfactorily used in the practice of the invention but the reaction may be conducted under reduced pressure or under pressure.

By this oxidation the disulfide bonds in the keratin material are converted into sulfo groups ($-SO_3H$).

(2) Derivatives of the mercapto groups of the products obtained by reduction of keratin materials.

Reducing agents employed for reducing keratin materials are preferably organic or inorganic reducing agents of the type which serves to cleave the disulfide bond in the keratin structure into a mercapto group (—SH) and generally act nucleophilically on the disulfide bond. Examples of the reducing agents include organic reducing agents such as 2-mercaptoethanol, thioglycollic acid, benzyl mercaptan, 1,4- dithiothreitol and tributylphosphine, and inorganic reducing agents such as sodium hydrogen sulfite, sulfides such as sodium hydrosulfide, metallic hydrides such as lithium aluminium hydride. The amount of the reducing agent is usually in the range of 2—10 equivalents of the disulfide bonds in keratin material. The pH of the reaction system is in the range of 2—12, preferably 6—11. Outside this range, undesired hydrolysis takes place at the same time. Room temperature is sufficient for the reaction but heat may be applied to shorten the reaction time. The reaction time is ordinarily in the range of 2—3 hours or more. Since the mercapto group produced by the reduction should not be substantially oxidized, the reduction should preferably be carried out in an atmosphere of an inert gas to give good results.

The mercapto group of the product obtained by the reduction of the keratin material is then chemically modified to yield a derivative thereof. The derivatives of the mercapto group include:

$-SCH_2COOH$, $-SCH_2CH_2COOH$, 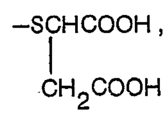 ,

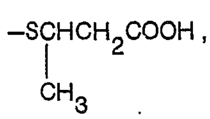 , 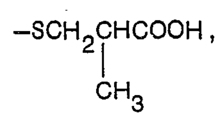 ,

$-SSO_3H$, $-SCH_2CH_2SO_3H$, 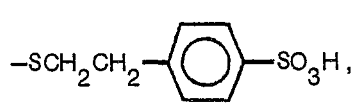 ,

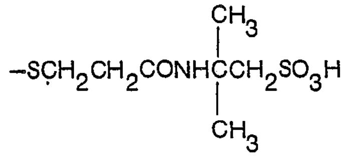 and $-SCH_2CH_2SO_2CH_2COOH$,

among which $-SCH_2COOH$ and 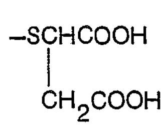 are preferred.

The chemical modification of the mercapto group is known per se and can be conducted, for example, based on the procedures known from N. H. Leon; Textile Progress, Vol. 7, page 1 (1975), "Yuki loo Kagobutsu (Organic Sulfur Compounds)" written by Shigeru Ookyo and published by Kagaku Dojin (1968) and "Kobunshi Jikkengaku Koza" written by Masami Oku, Vol. 12, Kyoritsu Shuppan (1957). Typical methods are shown below.

(a) Method utilizing the nucleophilic substitution reaction of SH group.

$$K—SH + R—L \quad K—S—R + HL$$

(in which K represents a residue of a keratin compound, R represents a chemically modifying group to be introduced, and L represents a leaving atom or group such as a halogen atom or acid residue).

Compounds which react by this method include, for example, halogen compounds such as iodoacetic acid, bromoacetic acid and chloroacetic acid.

(B) Method utilizing the nucleophilic addition reaction of SH group with a carbon-carbon double bond

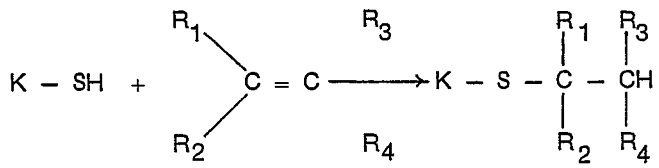

(in which at least one of $R_1$, $R_2$, $R_3$ and $R_4$ represents a carboxyl group or sulfo group, the other represent an alkyl group or hydrogen atom, and K has the same meaning as defined hereinbefore).

Compounds which react by this method include, for example, acrylic acid, methacrylic acid, crotonic acid, maleic acid, fumaric acid, vinyl carboxymethyl sulfone, vinylsulfonic acid, p-styrenesulfonic acid and 2-acrylamido-2-methyl-propanesulfonic acid.

(c) Method using a substitution reaction between SH group and sulfite compound

$$K - SH + NaHSO_3 \longrightarrow K - S - SO_3H$$

$$K - SH + Na_2SO_3 \xrightarrow[Air]{} K - S - SO_3H$$

(in which K has the same meaning as defined hereinbefore).

The derivatives of the keratin material used in the present invention are originally insoluble in polar solvents such as water, ethanol, propylene glycol and the like but show solubility in the presence of anionic surface active agents. In order to further increase the solubility, it is desirable to use bases in combination or for forming salts with the derivative. Examples of the bases include inorganic bases compounds such as sodium hydroxide and potassium hydroxide and organic bases such as ammonia, ethanolamine, diethanolamine, triethanolamine, 2-amino-2-methylpropanol, triisopropanolamine, glycine, arginine and histidine.

The shampoo composition according to the invention is preferably prepared by adding 0.05—10 wt% (hereinafter referred to simply as %), preferably 0.2—3%, of one or more of the derivatives of keratin material to a shampoo base containing 5—30%, preferably 10—25%, of one or more of anionic surface active agents. Lower amounts of the derivative than 0.1% are not suitable since the satisfactory effects are not shown, whereas larger amount than 10% are unsuitable since hair becomes sticky under high humidity.

Preferably, the pH of the shampoo composition of the invention is adjusted so that in a 5% aqueous solution the pH is in the range of 4—8.

The shampoo composition of the invention may further comprise, aside from the above-described two essential components, ingredients added to known shampoo compositions. For instance, there may be added, if necessary, the following ingredients in such amounts as not to impair the effect of the invention: amphoteric surface active agents, nonionic surface active agents, cationic surface active agents, solubilizing agents such as propylene glycol, glycerine and urea, viscosity-adjusting agents such as ethyl alcohol, isopropyl alcohol, hydroxyethyl cellulose, methyl cellulose and higher alcohols, perfumes, colorants, UV absorbers, antioxidants, preservatives, pearling agents, and lotioning agents.

The thus obtained shampoo composition of the invention has not only excellent hair conditioning effect and washing effect, but also shows milder eye irritation than known shampoo compositions and only a mild effect on the conjuctiva and iris.

The present invention is particularly described by way of references and examples, which should not be construed as limiting the present invention thereto.

Reference 1

Preparation of oxidation decomposition derivatives of keratin materials:

(a) Ten grams of wool fibers were oxidised by immersing them in 700 g of 8% aqueous peracetic acid solution at room temperature for 1 day. The resulting oxidized wool was filtered and washed with water, and then immersed in 700 g of a 0.1N ammonia solution at room temperature for 1 day, about 90% of the wool dissolved in the ammoniacal solution. About 1 g of the insoluble material was removed by filtration and the pH of the aqueous ammoniacal solution of keratose (oxidised product of wool keratin) was adjusted with 2N hydrochloric acid to 4.0 whereupon α-keratose precipitated. This precipitate was filtered, washed with acetone and dried to yield 5.4 g of α-keratose.

(b) Wool fibers were heated under pressure in an autoclave by the use of saturated steam at 6 $kg/cm^2$ for 6 minutes and were abruptly released into the atmosphere to yield a porous swollen product. Ten grams of the swollen product, which had been reduced to pieces, were immersed in 250 g of formic acid and 50 g of a 30% aqueous hydrogen peroxide solution in a 500 ml three neck distillation flask at room temperature for 1 day, whereupon no powder was found in the solution but a foam-like matter floated on the upper layer. This reaction mixture was filtered and the filtrate was poured into 1.5 liters of water, followed by adding hydrochloric acid to adjust the pH to 4. The resulting precipitate was collected by filtration and washed with 500 ml of water to yield 4.5 g of α-keratose. To the insoluble material from which the reaction product had been removed by filtration in 350 ml of water was added an aqueous ammonia solution to adjust the pH to 11, and the material was left to stand at room temperature for 1 day. The reaction mixture was filtered and hydrochloric was added to the filtrate to adjust the pH to 4. The resulting precipitate was collected by filtration to yield 0.7 g of α-keratose. It was found that insoluble material (1.4 g) was primarily β-keratose.

Reference 2

Preparation of reduced derivatives of keratin materials:

(a) To ten grams of wool fibers immersed in 600 ml of an aqueous solution containing 8 M urea and 0.01 M Tris buffer was added 6 ml of 2-mercaptoethanol, followed by adjusting the pH to 10 by means of aqueous 5N potassium hydroxide. The reduction reaction was carried out in an atmosphere of nitrogen at room temperature. About 3 hours after commencement of the reaction, 85% of the wool had dissolved in the reaction solution. While adjusting the pH of the reaction mixture with aqueous 5N potassium hydroxide so that the pH did not fall below 7, 16.5 g of iodoacetic acid was gradually added and the pH of the system was finally adjusted to 8.5. The carboxymethylation reaction was carried out at room temperature for 2 hours. The reaction solution was filtered to remove insoluble material therefrom and the resultant filtrate was charged into a cellulose tube wherein it was dialyzed against deionized water to remove low molecular weight impurities including urea. As the urea was dialyzed, the content in the cellulose tube turned white since water-insoluble HGT (component with high contents of glycine and tyrosine) precipitated. After completion of the dialysis, the HGT was removed by centrifugal separation and S-carboxymethylkerain (SCMKA) was obtained from the neutral transparent aqueous solution of SCMKA by the isoelectric precipitation. That is, 1N hydrochloric acid was added to the mixture to adjust its pH to 4.4 when SCMKA precipitated. This precipitate was filtered, washed with ethanol and dried to obtain 4.2 g of SCMKA.

(b) The procedure of Reference 2—(a) was repeated except that feathers were used instead of wool fibers. The feathers were heated for 6 minutes in an autoclave by means of superheated steam at 6 $kg/cm^2$ and 240°C and then abruptly released into the atmosphere to obtain a porous swollen product and that 1.75 g of maleic acid was used instead of iodoacetic acid, thereby obtaining 5.3 g of S-(1,2-dicarboxyethyl)-keratin.

(c) The procedure of Reference 2—(a) was repeated using powdered horses' hooves instead of wool fibers and 11 g of acrylic acid instead of iodoacetic acid, thereby obtaining 4.2 g of S-(2-carboxyethyl)-keratin.

(d) The procedure of Reference 2—(a) was repeated using 28 g of p-styrenesulfonic acid instead of iodoacetic acid, thereby obtaining 4.8 g of S-(p-sulfophenylethyl)-keratin.

(e) Eight grams of wool fibers were dispersed in 300 ml of n-propanol and 300 ml of a 0.1N Tris buffer solution. after displacing the air with nitrogen, 3.2 ml of tri-n-butylphosphine was added, followed by agitating at room temperature for 24 hours. The solution was filtered and to the insoluble material were added 400 ml of water, 9.28 g of maleic acid and about 30 ml of 5N potassium hydroxide to adjust the pH to 8.0, after which it was agitated at room temperature for 6 hours. To the reaction mixture was added about 20 ml of a 28% aqueous ammonia to adjust the pH to 11.5, after which it was agitated at room temperature for 18 hours. The reaction mixture was filtered to remove insoluble material therefrom and the resultant filtrate was placed in a cellulose tube in which it was dialyzed against deionized water to remove low molecular weight impurities therefrom. After completion of the dialysis, the insoluble material in the cellulose tube was removed by centrifugal separation and the neutral transparent aqueous solution was adjusted to a pH of 4.4 by addition of about 5.5 ml of 1N hydrochloric acid and the resulting precipitate was collected by filtration, followed by washing with ethanol and drying to obtain 3.9 g of S-(1,2-dicarboxyethyl)-keratin.

(f) The procedure of Reference 2—(e) was repeated except that a powdered porous swollen product obtained by heating wool in an autoclave by means of saturated steam at 6 $kg/cm^2$ for 6 minutes was used

instead of wool fibres and that 16.5 g of 2-acrylamido-2-methylpropanesulfonic acid was used instead of maleic acid, thereby obtaining 4.5 g of S-[2-(1,1-dimethyl-2-sulfoethylcarbamoyl)ethyl]-keratin.

Example 1

Shampoo compositions containing 30% of coconut fatty acid diethanolamide and suitable amounts of perfumes, and anionic surface active agents and derivatives of keratin materials indicated in Table 1 were prepared to evaluate their performance. The results are shown in Table 2.

The evaluation tests were conducted according to the following methods.

(1) Lathering test

A 1% aqueous solution of each shampoo composition was artificially stained with 0.1% of lanolin. The composition was agitated in a cylinder by means of a plain propeller at a rate of 1000 r.p.m. at 40°C for 5 minutes under such conditions that the rotation was reversed every 10 seconds. Thirty seconds after completion of the agitation, the amount of lather was measured.

(2) Feel of Lather

After thirty grams of a human hair had been wetted with water at 40°C, the excess water was squeezed out to leave 20 g of the water in the hair. Then, the hair was washed using 1 g of each shampoo composition and the feel of the lather was sensorially evaluated by a panel of twenty females.

Evaluation Items:

The degree of ease of passing fingers through hair while washing was evaluated as "slipping of lather" and the appearance of the lather was evaluated from a viewpoint of "creaminess".

Evaluation Standards:

○ The slipping of lather is better than that of reference product or the appearance of lather is more creamy.

△ Slightly better than reference product.

× Equal to reference product.

Reference Product:

| | |
|---|---|
| Sodium polyoxyethylene (3) laurylsulfate | 15% |
| Coconut fatty acid diethanolamide | 3 |
| Perfume | suitable amount |
| Water | balance (pH 7.2) |

(3) Combing Force

After thirty grams of artificial hair was wetted with water at 40°C, the excess water was squeezed out to leave 20 g of water in the hair. One gram of each shampoo composition was used for washing the hair and a rinsing operation was repeated twice. The hair was attached to a strain gauge after the excess water was squeezed out and combed to measure the force exerted on the hair at the time of the combing (wet state). Then, the hair was dried by a dryer and allowed to stand overnight in an air-conditioned room of 20°C and 65% R.H. and then attached to a strain gauge, followed by combing to measure the force exerted on the hair at the time of combing (dry state).

(4) Hair Fly

When measuring the "combing force" in the dry state, it was observed whether or not the hair fly phenomenon occurred by the action of static electricity.

Evaluation:

× Hair fly took place.

△ Hair fly took place slightly.

○ Hair fly did not take place.

TABLE I

| Sample No. | Anionic Surface Active Agent | Derivative of Keratin Material |
|---|---|---|
| 1 | triethanolamine lauryl sulfate (15%) | Derivative of reference 2–(a) 2% |
| 2 | ,, | ,, reference 2–(b) 2% |
| 3 | ,, | ,, reference 2–(c) 2% |
| 4 | ,, | ,, reference 2–(d) 2% |
| 5 | ,, | ,, reference 2–(e) 2% |
| 6 | ,, | ,, reference 2–(f) 2% |
| 7 | sodium polyoxyethylene (2) lauryl sulfate (15%) | ,, reference 2–(a) (0.1%) |
| 8 | ,, | ,, ,, (1.0%) |
| 9 | ,, | ,, ,, (10%) |
| 10 | ,, | ,, ,, (20%) |
| 11 | triethanolamine lauryl sulfate (15%) | acid decomposition product of collagen (M.W. 10,000–20,000) (2%) |
| 12 | ,, | Alkali decomposition product of collagen (M.W. 800–1,000) (3%) |
| 13 | ,, | nil |

TABLE 2

| Sample No. | Lathering Characteristics | | | Characteristics after Washing | | |
|---|---|---|---|---|---|---|
| | Amount of Lather (ml) | Feel of Lather | | Combing force (g) | | Hair fly |
| | | Slipping of lather | Creaminess | Wet st. | Dry st. | |
| 1 | 170 | o | o | 190 | 80 | o |
| 2 | 165 | o | o | 180 | 75 | o |
| 3 | 160 | o | o | 220 | 90 | o |
| 4 | 165 | o | o | 205 | 90 | o |
| 5 | 167 | o | o | 200 | 85 | o |
| 6 | 165 | o | o | 205 | 85 | o |
| 7 | 135 | o~Δ | o~Δ | 295 | 160 | o~Δ |
| 8 | 151 | o | o | 220 | 110 | o |
| 9 | 162 | o | o | 195 | 95 | o |
| 10 | 159 | o | o | 200 | 95 | o |
| 11 | 152 | Δ | Δ | 350 | 175 | Δ |
| 12 | 148 | Δ | Δ | 360 | 170 | Δ |
| 13 | 112 | x | x | 470 | 210 | x |

Example 2

Shampoo compositions A (inventive product) and B (comparative product) of the following formulations were prepared and their effects were evaluated by a paired sensorial evaluation by a panel of 20 females. The results are shown in Table 3.

Shampoo composition:

| | A | B |
|---|---|---|
| Triethanolamine lauryl sulfate | 18.0 (%) | 18.0 (%) |
| Derivative of keratin material (obtained in reference 2—(a)) | 2.0 | — |
| Perfume | 0.3 | 0.3 |
| Colorant | Small amount | Small amount |
| Water | Balance | Balance |

TABLE 3

| Evaluation Item | Shampoo A being better | Shampoo B being better | Hard to say |
|---|---|---|---|
| Creaminess of lather | 15 | 3 | 2 |
| Ease of passing fingers through hair | 17 | 1 | 2 |
| Feel of hair after washing (wet) | 17 | 0 | 3 |
| Feel of hair after washing (dry) | 19 | 0 | 1 |
| Ease of setting hair | 18 | 0 | 2 |

Example 3

The shampoo composition A (inventive product) used in Example 2 and a commercially available shampoo were used to check the degree of irritation to the eyes by the Draize method (see Cosmetics and the Skin; F. V. Wells and I. I. Lubowe, Reinhold Publishing Corpn.). That is 0.1 ml of each shampoo composition was dropped into the eyes of five healthy white, male rabbits weighing 2.5—3.0 kg. Three hours and 24 hours after the application, the cornea, iris and conjuctiva of the five rabbits were observed and the results were calculated according to the following inspection method to give average values. A total value was obtained by adding a value which was obtained by multiplying two average values for the cornea by themselves and further by 5, a value which was obtained by multiplying an average value for the iris by 5, and a value which was obtained by adding three average values for the conjuctiva to one another and then multiplying by 2. A higher total value shows a greater degree of the irritation to eyes. The results are shown in Table 4.

Inspection Method:

1. Cornea:

   Opacity 0∾4

   Area of cornea involved 0∾4

2. Iris:

   Congestion 0∾2

3. Conjuctiva:

   Redness 0∾3

   Chemosis 0∾4

   Discharge 0∾3

TABLE 4

| Shampoo Composition | | A (Inventive product) | | Commercially available product | |
|---|---|---|---|---|---|
| Time after dropping (h) | | 3 | 24 | 3 | 24 |
| Cornea | Opacity | 1 | 0 | 1 | 1 |
| | Area of cornea involved | 1 | 0 | 4 | 2 |
| Iris | Congestion | 0 | 0 | 0 | 0 |
| Conjuctiva | Redness | 1 | 0 | 2 | 1 |
| | Chemosis | 0 | 0 | 2 | 1 |
| | Discharge | 1 | 0 | 2 | 1 |
| Total | | 9 | 0 | 32 | 16 |

**Claims for the Contracting States: BE CH DE FR IT LI NL**

1. A shampoo composition comprising a shampoo base containing at least one anionic surface active agent and a hair conditioning derivative of keratin material selected from (1) derivatives of the mercapto groups of the products obtained by reduction of keratin materials, and (2) a mixture thereof with oxidized keratin materials containing $—SO_3H$ groups.

2. A shampoo composition according to Claim 1, wherein said anionic surface active agent is contained in an amount of 5 to 30% by weight of said shampoo composition.

3. A shampoo composition according to Claim 2, wherein said anionic surface active agent is contained in an amount of 10 to 25% by weight of said shampoo composition.

4. A shampoo composition according to Claim 1, 2 or 3, wherein said keratin derivative is contained in an amount of 0.05 to 10% by weight of said shampoo composition.

5. A shampoo composition according to Claim 4, wherein the amount of said keratin derivative is in the range of 0.2 to 3% by weight of the said shampoo composition.

6. A shampoo composition according to Claim 1, wherein the pH of said shampoo composition is adjusted so that in a 5% aqueous solution the pH is in the range of 4-8.

7. A shampoo composition according to Claim 1, wherein said anionic surface active agent is a linear or branched alkylsulfate having an average of 10 to 16 carbon atoms, a polyoxyethylenealkylsulfate whose alkyl grcup has an average of 8 to 20 carbon atoms or an olefin-sulfonate having an average of 10 to 20 carbon atoms.

8. A shampoo composition according to Claim 1, wherein the derivative of the mercapto group is selected from

$-SCH_2COOH$, $-SCH_2CH_2COOH$, 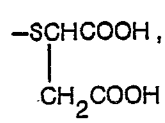 ,

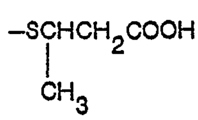 , 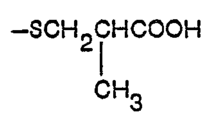 ,

$-SSO_3H$, $-SCH_2CH_2SO_3H$, 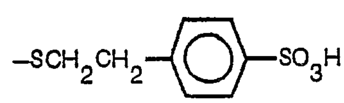 ,

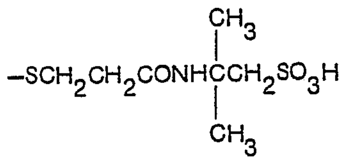 and $-SCH_2CH_2SO_2CH_2COOH$,

9. A method of treating hair which comprises applying thereto a shampoo composition as claimed in any one of claims 1 to 8.

**Claims for the contracting State: AT**

1. A method for the production of a shampoo composition comprising admixing a shampoo base containing at least one anionic surface active agent with a derivative of keratin material selected from (1) derivatives of the mercapto groups of the products obtained by reduction of a keratin material and (2) a mixture thereof with oxidised keratin material containing —$SO_3H$ groups.

2. A method according to Claim 1, wherein said anionic surface active agent is contained in an amount of 5 to 30% by weight of said shampoo composition.

3. A method according to Claim 2, wherein said anionic surface active agent is contained in an amount of 10 to 25% by weight of said shampoo composition.

4. A method according to Claim 1, 2 or 3 wherein said keratin derivative is contained in an amount of 0.05 to 10% by weight of said shampoo composition.

5. A method according to Claim 4, wherein the amount of said keratin derivative is in the range of 0.2 to 3% by weight of said shampoo composition.

6. A method according to Claim 1, wherein the pH of said shampoo composition is adjusted so that in a 5% aqueous solution the pH is in the range of 4-8.

7. A method according to any preceding Claim wherein said anionic surface active agent is a linear or branched alkylsulfate having an average of 10 to 16 carbon atoms, a polyoxyethylenealkylsulfate whose alkyl group has an average of 8 to 20 carbon atoms, or an olefinsulfonate having an average of 10 to 20 carbon atoms.

8. A method according to any preceding claim, wherein the derivative of the mercapto group is selected from

$-SCH_2COOH$, $-SCH_2CH_2COOH$, 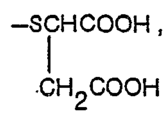,

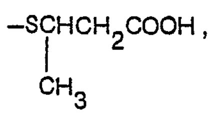, 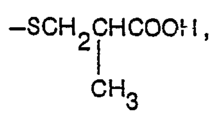,

$-SSO_3H$, $-SCH_2CH_2SO_3H$, 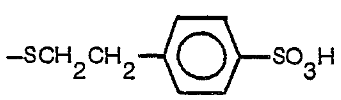,

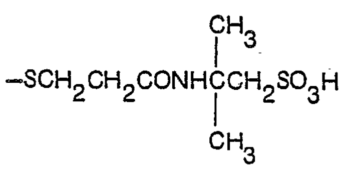 and $-SCH_2CH_2SO_2CH_2COOH$,

9. A method of treating hair which comprises applying thereto a shampoo composition produced by the method of any of claims 1 to 8.

**Patentansprüche für die Vertragsstaaten: BE CH DE FR IT LI NL**

1. Haarwaschmittel, umfassend eine Shampoobasis, enthalten mindestens ein anionisches oberflächenaktives Mittel, und ein Haarkonditionierungs-Derivat aus Keratinmaterial, ausgewählt aus

1) Derivaten der Mercaptogruppen von Produkten, die durch Reduktion von Keratinmaterialien erhalten wurden, und

2) einem Gemisch derselben mit oxidierten Keratinmaterialien, welche $SO_3H$-Gruppen enthalten.

2. Haarwaschmittel nach Anspruch 1, wobei das anionische oberflächenaktive Mittel in einer Menge von 5 bis 30 Gew.% des Haarwaschmittels enthalten ist.

3. Haarwaschmittel nach Anspruch 2, wobei das anionische oberflächenaktive Mittel in einer Menge von 10 bis 25 Gew.% des Haarwaschmittels enthalten ist.

4. Haarwaschmittel nach Anspruch 1, 2 order 3, wobei das Keratinderivat in einer Menge von 0,05 bis 10 Gew.% des Haarwaschmittels enthalten ist.

5. Haarwaschmittel nach Anspruch 4, wobei die Menge des Keratinderivats im Bereich von 0,2 bis 3 Gew.% des Haarwaschmittels beträgt.

6. Haarwaschmittel nach Anspruch 1, wobei der pH des Haarwaschmittels derart eingestellt ist, daß in einer 5%igen wässrigen Lösung der pH im Bereich von 4 bis 8 liegt.

7. Haarwaschmittel nach Anspruch 1, wobei das anionische oberflächenaktive Mittel ein lineares oder verzweigtes Alkylsulfat mit durchschnittlich 10 bis 16 Kohlenstoffatomen, ein Polyoxyethylenalkylsulfat, dessen Alkylgruppe durchschnittlich 8 bis 20 Kohlenstoffatome aufweist, oder ein Olefinsulfonat mit durchschnittlich 10 bis 20 Kohlenstoffatomen ist.

8. Haarwaschmittel nach Anspruch 1, wobei das Derivat der Mercaptogruppe ausgewählt ist aus

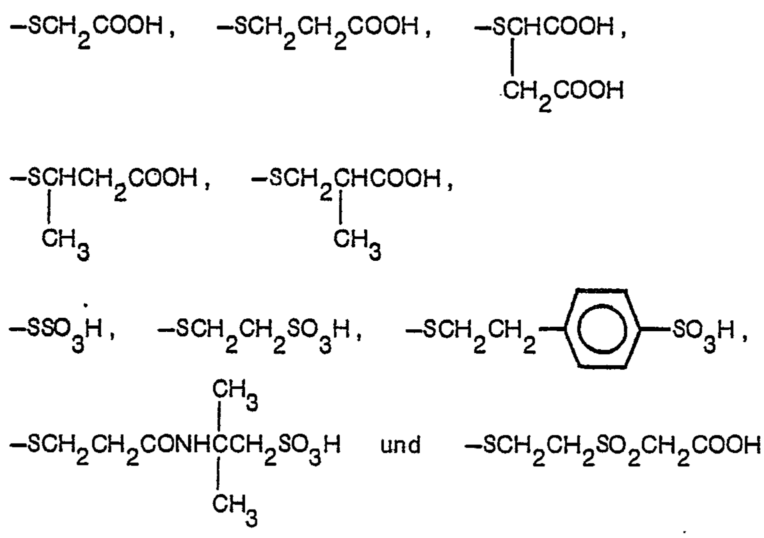

und

9. Verfahren zur Haarbehandlung, umfassend die Applikation eines Haarwaschmittels gemäß einem der Ansprüche 1 bis 8 auf das Haar.

**Patentansprüche für den Vertragsstaat: AT**

1. Verfahren zur Herstellung eines Haarwaschmittel, umfassend das Vermischen einer Shampoobasis, enthaltend mindestens ein anionisches oberflächenaktives Mittel, mit einem Derivat von Keratinmaterial, ausgewählt aus

1) Derivaten der Mercaptogruppen von Produkten, die durch Reduktion eines Keratinmaterials erhalten wurden, und

2) einem Gemisch derselben mit oxidiertem Keratinmaterial, das $SO_3H$-Gruppen enthält.

2. Verfahren nach Anspruch 1, wobei das anionische oberflächenaktive Mittel in einer Menge von 5 bis 30 Gew.% des Haarwaschmittels enthalten ist.

3. Verfahren nach Anspruch 2, wobei das anionische oberflächenaktive Mittel in einer Menge von 10 bis 25 Gew.% des Haarwaschmittels enthalten ist.

4. Verfahren nach Anspruch 1, 2 oder 3, wobei das Keratinderivat in einer Menge von 0,05 bis 20 Gew.% des Haarwaschmittels enthalten ist.

5. Verfahren nach Anspruch 4, wobei die Menge des Keratinderivats im Bereich von 0,2 bis 3 Gew.% des Haarwaschmittels beträgt.

6. Verfahren nach Anspruch 1, wobei der pH des Haarwaschmittels derart eingestellt ist, daß in einer 5%igen wässrigen Lösung der pH im Bereich von 4 bis 8 liegt.

7. Verfahren nach einem der vorstehenden Ansprüche, wobei das anionische oberflächenaktive Mittel ein lineares oder verzweigtes Alkylsulfat mit durchschnittlich 10 bis 16 Kohlenstoffatomen, ein Polyoxyethylenalkylsulfat, dessen Alkylgruppe durchschnittlich 8 bis 20 Kohlenstoffatome aufweist, oder ein Olefinsulfonat mit durchschnittlich 10 bis 20 Kohlenstoffatomen ist.

8. Verfahren nach einem der vorstehenden Ansprüche, wobei das Derivat der Mercaptogruppe ausgewählt ist aus

$-SCH_2COOH$, $-SCH_2CH_2COOH$, 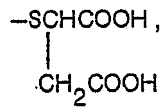,

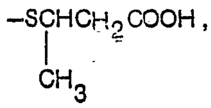, 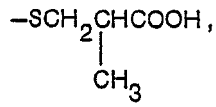,

$-SSO_3H$, $-SCH_2CH_2SO_3H$, 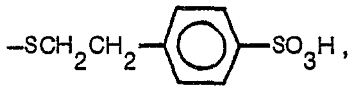,

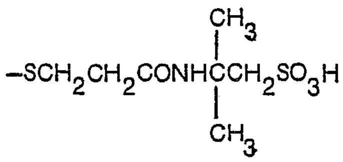 und $-SCH_2CH_2SO_2CH_2COOH$.

9. Verfahren zur Haarbehandlung, wobei man dem Haar ein Haarwaschmittel appliziert, das nach dem Verfahren eines der Ansprüche 1 bis 8 hergestellt wurde.

**Revendications pour les Etats Contractants: BE CH DE FR IT LI NL**

1. Composition de shampooing comprenant une base de shampooing contenant au moins un agent tensio-actif anionique et un dérivé d'un matériau en kératine pour le conditionnement des cheveux choisi parmi (I) des dérivés des groupes mercapto des produits obtenus par réduction des matériaux en kératine, et (2) un mélange de ces dérivés avec des matériaux en kératine oxydés contenant des groupes $-SO_3H$.

2. Composition de shampooing selon la revendication 1, dans laquelle ledit agent tensio-actif anionique est contenu en une quantité de 5 à 30 % en poids de ladite composition de shampooing.

3. Composition de shampooing selon la revendication 2, dans laquelle ledit agent tensio-actif anionique est contenu en une quantité de 10 à 25 % en poids de ladite composition de shampooing.

4. Composition de shampooing selon la revendication 1, 2 ou 3, dans laquelle ledit dérivé de la kératine est contenu en une quantité de 0,05 à 10 % en poids de ladite composition de shampooing.

5. Composition de shampooing selon la revendication 4, dans laquelle la quantité dudit dérivé de la kératine est comprise dans la plage allant de 0,2 à 3 % en poids de ladite composition de shampooing.

6. Composition de shampooing selon la revendication 1, dans laquelle le pH de ladite composition de shampooing est réglé de sorte que dans une solution aqueuse à 5 % le pH soit dans la plage allant de 4 à 8.

7. Composition de shampooing selon la revendication 1, dans laquelle ledit agent tensio-actif anionique est un alkylsulfate linéaire ou ramifié possédant une moyenne de 10 à 16 atomes de carbone, un polyoxyéthylènealkylsulfate dont le groupe alkyle possède une moyenne de 8 à 20 atomes de carbone ou un oléfinesulfonate possèdant une moyenne de 10 à 20 atomes de carbone.

8. Composition de shampooing selon la revendication 1, dans laquelle le dérivé du groupe mercapto est choisi parmi $-SCH_2COOH$,

$-SCH_2COOH$, $-SCH_2CH_2COOH$, 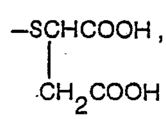,

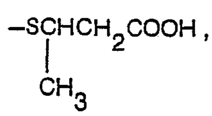, 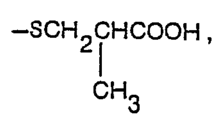,

$-SSO_3H$, $-SCH_2CH_2SO_3H$, 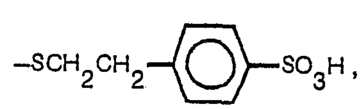,

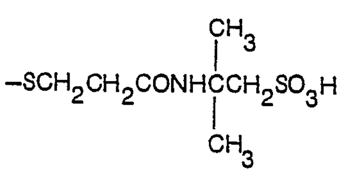 et $-SCH_2CH_2SO_2CH_2COOH$,

9. Procédé de traitement des cheveux qui consiste à leur appliquer une composition de shampooing telle que revendiquée dans l'une quelconque des revendications 1 à 8.

**Revendications pour l'Etat contractant: AT**

1. Procédé pour la production d'une composition de shampooing, consistant à mélanger une base de shampooing contenant au moins un agent tensio-actif anionique avec un dérivé d'un matériau en kératine choisi parmi (1) des dérivés des groupes mercapto des produits obtenus par réduction d'un matériau en kératine et (2) un mélange de ces dérivés avec des matériaux en kératine oxydés contenant des groupes $-SO_3H$.

2. Procédé selon la revendication 1, dans lequel ledit agent tensio-actif anionique est contenu en une quantité de 5 à 30 % en poids de ladite composition de shampooing.

3. Procédé selon la revendication 2, dans lequel ledit agent tensio-actif anionique est contenu en une quantité de 10 à 25 % poids de ladite composition de shampooing.

4. Procédé selon la revendication 1, 2 ou 3, dans lequel ledit dérivé de la kératine est contenu en une quantité de 0,05 à 10 % en poids de ladite composition de shampooing.

5. Procédé selon la revendication 4, dans lequel la quantité dudit dérivé de la kératine est comprise dans la plage allant de 0,2 à 3 % en poids de ladite composition de shampooing.

6. Procédé selon la revendication 1, dans lequel le pH de ladite composition de shampooing est réglé de sorte que dans une solution aqueuse à 5 % le pH soit dans la plage allant de 4 à 8.

7. Procédé selon l'une quelconque des revendications précédentes, dans lequel ledit agent tensio-actif anionique est un alkylsulfate linéaire ou ramifié possédant une moyenne de 10 à 16 atomes de carbone, un polyoxyéthylènealkylsulfate dont le groupe alkyle possède une moyenne de 8 à 20 atomes de carbone ou un oléfinesulfonate possèdant une moyenne de 10 à 20 atomes de carbone.

8. Procédé selon l'une quelconque des revendications précédentes, dans lequel le dérivé du groupe mercapto est choisi parmi

$-SCH_2COOH$, $-SCH_2CH_2COOH$, 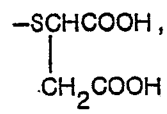

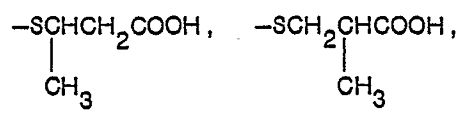

$-SSO_3H$, $-SCH_2CH_2SO_3H$, 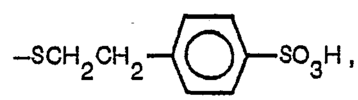

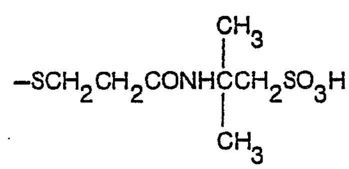 et $-SCH_2CH_2SO_2CH_2COOH$,

9. Procédé de traitement des cheveux qui consiste à leur appliquer une composition de shampooing préparée par le procédé selon l'une quelconque des revendications 1 à 8.